# EUROPÄISCHE PATENTSCHRIFT

(11) **EP 1 776 353 B1**
(45) Veröffentlichungstag und Bekanntmachung des Hinweises auf die Patenterteilung: **02.01.2013**
(21) Anmeldenummer: 05777969.6
(22) Anmeldetag: 03.08.2005
(51) Int. Cl.: C07D 295/02

(54) **VERFAHREN ZUR VERBESSERUNG DER FARBSTABILITÄT VON PIPERAZIN**
METHOD FOR IMPROVING COLOUR STABILITY OF PIPERAZINE
PROCEDE D'AMELIORATION DE LA STABILITE DES COULEURS DE LA PIPERAZINE

(30) Priorität: 05.08.2004 DE 102004038107
(43) Veröffentlichungstag der Anmeldung: 25.04.2007
(73) Patentinhaber: BASF SE, 67056 Ludwigshafen (DE)
(72) Erfinder: EVERS, Holger, 67063 Ludwigshafen (DE); JÖDECKE, Michael, 67240 Bobenheim-Roxheim (DE); MELDER, Johann-Peter, 67459 Böhl-Iggelheim (DE); VAN CAUWENBERGE, Gunther, 9140 Temse (BE); NOUWEN, Jan, B-2960 Brecht (BE)
(86) Internationale Anmeldenummer: PCT/EP2005/008401
(87) Internationale Veröffentlichungsnummer: WO 2006/015780

(56) Entgegenhaltungen:
- GB-A- 1 263 588
- US-A- 3 105 019

## Beschreibung

Die vorliegende Erfindung betrifft ein Verfahren zur Verbesserung der Farbstabilität durch Nachreinigung von Piperazin.

Piperazin findet Anwendung bei der Herstellung einer breiten Palette von Pharma- und Tierpharmaprodukten bis hin zu Klebstoffen. Deshalb werden an Piperazin besondere Anforderungen hinsichtlich Reinheit und Qualität gestellt.

Ein wichtiges Qualitätsmerkmal neben der meist gaschromatographisch ermittelten Reinheit und dem Wassergehalt ist der Grad der Verfärbung und die Farbstabilität.

Als ein Maß für den Grad der Verfärbung wird meist die so genannte Farbzahl herangezogen. Die Farbzahl ist ein unter festgelegten Bedingungen ermittelter Kennwert für die Farbe von transparenten Substanzen, der durch optischen Vergleich festgestellt wird. Eine häufig verwendete Farbzahl bei Flüssigkeiten ist die APHA (American Public Health Association)-Farbzahl (Römpp Chemie Lexikon 1995).

Damit Piperazin in der gewünschten Reinheit erhalten werden kann, sind in der Literatur verschiedene Aufreinigungsverfahren beschrieben. So kann die Aufreinigung beispielsweise über die Stufe der Carbamate, Hydrochloride oder Imine erfolgen.

In DE-A-195 36 792 ist beispielsweise die Trennung und Aufreinigung durch Kristallisation beschrieben.

In modernen Verfahren erfolgt der Erhalt des Piperazins in gewünschter Reinheit jedoch meist bei der destillativen Abtrennung von einem Produktgemisch. Hierbei stehen mittlerweile derart leistungsstarke Destillationsapparaturen zur Verfügung, dass die letztendlich erhaltene Piperazinfraktion bereits den gewünschten Reinheitsgrad besitzt. Somit beinhaltet die Destillation sowohl den Verfahrensschritt der Abtrennung (Isolierung) als auch den der Reinigung in einem. Dies stellt aus ökonomischer Sicht ein besonders effizientes Vorgehen dar, auch wenn das Piperazin erst mehrfach in einer oder mehreren Kolonnen fraktioniert werden muß.

Im technischen Maßstab wird Piperazin meist bei der Herstellung verschiedener Ethylenamine als eines der Wertprodukte erhalten. Hierbei beruht die Synthese auf der Umsetzung von Ethylendichlorid (EDC-Verfahren) oder Monoethanolamin (MEOA-Verfahren) mit Ammoniak. Weitere Koppelprodukte dieser Umsetzung sind Ethylendiamin (EDA), Diethylentriamin (DETA), Triethylentetramin (TETA) und höhere lineare und cyclische Ethylenamine sowie zusätzlich Aminoethylethanolamin (AEEA) beim MEOA Verfahren.

Beide Syntheserouten basieren letztlich auf der Oxidation von Ethylen (mit Chlor oder Sauerstoff) zu EDC bzw. Ethylenoxid (EO) und anschließender ein- oder zweistufiger Umsetzung mit Ammoniak. Zwar beinhaltet das MEOA Verfahren damit einen zusätzlichen Syntheseschritt, das Oxidationsmittel Chlor ist jedoch teuer und zusätzlich ist ein Salzanfall unvermeidlich.

Im Vergleich zum EDC Verfahren gewährleistet das MEOA-Verfahren einen erhöhten Anteil an cyclischen Verbindungen, so dass dieses, wenn ein piperazinhaltiger Produktmix gewünscht wird, das bevorzugte Verfahren darstellt.

Unabhängig von der Wahl des Verfahrens erfolgt die Aufreinigung und Trennung des Ethylenamin-Produktmixes bei industrieller Produktion meist über eine Kaskade von Kolonnen in kontinuierlichem Betrieb. Zunächst wird dabei der Ammoniak in einer Druckkolonne abgezogen, danach erfolgt die Abdestillation des gebildeten (oder beim EDC Verfahren zugesetzten) Prozesswassers.

GB 1 263 588 beschreibt hierbei die Reinigung des Produktmixes durch das azeotrope Entfernen von Verunreinigungen beim Abdestillieren von Wasser.

US 3,105,019 beschreibt hingegen den Zusatz organischer Lösemittel als Schleppmittel für Piperazin in der Destillation speziell zur Abtrennung einer Piperazin-TEDA Fraktion.

Alle oben beschriebenen Verfahren liefern zwar Piperazin oder andere Ethylamin-Produkte oder -Produktgemische mit ausreichender Reinheit, jedoch ist nicht gewährleistet, dass die Produkte über eine ausreichende Farbstabilität verfügen, selbst wenn die ermittelte Farbzahl unmittelbar nach Isolierung den Anforderungen entspricht.

Dies liegt daran, dass die Verfärbungskomponenten in der Regel als Einzelverbindungen nicht faßbar sind und beispielsweise in der gaschromatographischen Reinheitskontrolle unter der Detektionsgrenze liegen. Insbesondere im MEOA-Verfahren basieren diese Verbindungen auf Acetaldehydfragmenten und deren kondensierten Folgeprodukten. Hierbei bilden sich die Chromophor-gebenden Verbindungen erst im Laufe der Zeit, so dass eine Farbzahländerung häufig erst nach Lagerung dahingehend eintritt, dass die gewünschte Spezifikation nicht mehr erreicht wird.

Der vorliegenden Erfindung liegt daher die Aufgabe zugrunde, ein Verfahren bereitzustellen, das die Farbstabilität von an und für sich ausreichend reinem Piperazin verbessert.

Die Aufgabe wird gelöst durch ein Verfahren zur Verbesserung der Farbstabilität durch Nachreinigung von Piperazin, die Schritte umfassend
- Herstellen einer wäßrigen Piperazin-Lösung durch Einleiten festen oder flüssigen Piperazins mit einem Reinheitsgrad von mindestens 90 Gew.-% in Wasser;
- Entfernen des Wassers durch Destillation;
- Isolierung des Piperazins mit einem vorgegebenen Höchstrestgehalt an Wasser.

Es wurde nämlich gefunden, dass durch diese Nachreinigung von Piperazin mit ausreichender Reinheit von 90 Gew.-%, wie sie leicht bei üblichen Destillationsverfahren erreicht werden kann, die Farbstabilität erhöht werden kann.

Das Verfahren dient insofern als Nachreinigung, da die üblichen Herstellverfahren, zum Beispiel mittels Destillation, bereits ein gereinigtes Produkt ergeben.

Hierbei wird zunächst flüssiges oder festes Piperazin in Wasser eingeleitet, um eine wäßrige Lösung zu erhalten. Das anschließende destillative Entfernen des Wasser vermag die Verunreinigungen als Azeotrop vom Piperazin abzutrennen, wodurch eine höhere Farbstabilität erreicht wird und somit die Piperazin Ware lagerfähiger ist.

Bei den so abgetrennten Verunreinigungen handelt es sich meist um erst später farbgebende Komponenten, die zum Zeitpunkt der Produktgewinnung in modernen Destillationsanlagen noch als farblose "Prä-Chromatophore" existieren, welche abhängig von Art und Dauer der Lagerung durch Kondensation und Oxidation zu einem nicht charakterisierbaren Gemisch farbiger Verbindungen reagieren. Auslösend für den Effekt der Vergilbung sind letztlich Kleinstmengen an Verunreinigungen, die im frisch isolierten Produkt durch Standardanalysemethoden nicht eindeutig nachweisbar sind.

Diese Verunreinigungen tauchen insbesondere im MEOA Verfahren auf, da die Aminierung von MEOA auf Ethyleneinheiten basiert, die die Bildung von C2-Fragmenten durch Dehydratisierung und Desaminierung unter den Reaktionsbedingungen ermöglichen. Diese Fragmente, speziell Acetaldehyd und höher kondensierte Folgeprodukte, verursachen hierbei eine nachhaltige Verschlechterung der Produktqualität, insbesondere der Farbstabilität. Auch hierbei gilt, dass in den isolierten Komponenten nach der Destillation diese Verunreinigungen jedoch aufgrund ihrer Inhomogenität und geringen Konzentration in der Regel analytisch nicht nachzuweisen sind.

Zur Bewertung der Farbstabilität des Produktes kann dann diese in Langzeitlagertests ermittelt werden.

Die Konzentration an in Wasser gelöstem Piperazin kann stark variiert werden. Zweckmäßigerweise liegt die Piperazin-Konzentration so hoch, dass kein Hexahydrat gebildet wird. Vorzugsweise liegt die Konzentration bei 50 Gew.-% oder mehr. Besonders bevorzugt zwischen 50 und 75 Gew.-%.

Die Herstellung des Piperazins kann nach verschiedenen Verfahren geschehen. Bevorzugt sind das EDC- und das MEOA Verfahren; besonders bevorzugt ist das MEOA Verfahren.

Bei diesen Verfahren beruht die Synthese auf der Umsetzung von Ethylendichlorid (EDC) oder Monoethanolamin (MEOA) mit Ammoniak. Weitere Koppelprodukte dieser Umsetzung sind - neben Piperazin - Ethylendiamin (EDA), Diethylentriamin (DETA), Triethylentetramin (TETA) und höhere lineare und cyclische Ethylenamine sowie zusätzlich Aminoethylethanolamin (AEEA) beim MEOA Verfahren.

Unabhängig von der Wahl des Verfahrens erfolgt die Aufreinigung und Trennung des Ethylenamin-Produktmixes bei industrieller Produktion meist über eine Kaskade von Kolonnen in kontinuierlichem Betrieb. Zunächst wird dabei der Ammoniak in einer Druckkolonne abgezogen, danach erfolgt die Abdestillation des gebildeten (oder beim EDC Verfahren zugesetzten) Prozesswassers.

Im Anschluss werden die verschiedenen Ethylenamine nach aufsteigenden Siedepunkten sortiert destillativ aufgetrennt (sortiert nach Siedepunkten EDA, Piperazin, MEOA, DETA, AEEA und weiter höhere). Alternativ können zunächst EDA und Piperazin gemeinsam abgetrennt und in einer nachgeschalteten Kolonne in die Einzelkomponenten aufgetrennt werden. In dieser werden EDA über Kopf und Piperazin im Sumpf oder als Seitenabzug erhalten.

Ein solcher Kolonnenaufbau kann so optimiert gefahren werden, dass die Einzelkomponenten in hohen Reinheiten isoliert werden.

Üblicherweise entspricht der Reinheitsgrad des Piperazins mehr als 90 Gew.-%, vorzugsweise mehr als 95 Gew.-%, besonders bevorzugt mehr als 99 Gew.-% und am meisten bevorzugt mehr als 99,9 Gew.-%, bevor dieses im erfindungsgemäßen Verfahren eingesetzt wird.

Somit wird das Piperazin vorzugsweise aus der destillativen Abtrennung eines Ethylenamin Produktmixes oder einer EDA/Piperazin-Fraktion erhalten und dem erfindungsgemäßen Verfahren zugeführt. Das Piperazin kann direkt als Fraktionsaustrag in Wasser eingeleitet werden (beispielsweise als 65 %-ige Lösung) oder vorher als Chips-Ware vorliegen. Bei der Chips-Ware handelt es sich um abgeschupptes Piperazin. Die Herstellung kann durch Abkühlen von aus der Piperazin Schmelze stammenden Tropfen erfolgen, die beispielsweise auf ein Band fallen und dabei in "Schuppen-Form" erstarren.

Die so gewonnenen frischen Chips können in dem erfindungsgemäßen Verfahren eingesetzt werden. Ebenso kann bereits gelagerte und gegebenenfalls bereits vergilbte Ware in Chips- oder anderer Form eingesetzt werden.

Das nach destillativem Entfernen des Wassers isolierte Piperazin sollte einen Höchstwasseranteil von unter 15 Gew.-%, bevorzugt unter 5 Gew.-% und besonders bevorzugt unter 1 Gew.-% aufweisen.

Bevorzugt werden zum Entfernen des Wassers eine oder mehrere Kolonnendestillationen durchgeführt, wobei das Wasser über den Kolonnenkopf entfernt wird.

Vorzugsweise erfolgt eine mehrstufige destillative Trennung. Die wäßrige Piperazin-Lösung wird in einer Destillationseinheit in hochreines Piperazin am Sumpf der Kolonne und Wasser mit organischen Bestandteilen am Kopf der Kolonne aufgearbeitet.

Ebenso kann das Piperazin über einen Seitenabzug der Aufreinigungskolonne isoliert werden.

Als Destillationseinheit kann eine Kolonne bekannter Bauart mit Verdampfer und Kondensator verwendet werden. Der Zulauf erfolgt bevorzugt in der oberen Hälfte der Kolonne, die Sumpftemperatur beträgt bevorzugt 130 bis 170 °C, der Druck am Kolonnenkopf vorzugsweise 0,6 bis 2 bar. Als Kolonneneinbauten kommen prinzipiell alle gängigen Einbauten in Betracht, beispielsweise Böden, Packungen oder Schüttungen. Von den Böden sind Glockenböden, Siebböden, Ventilböden, Thormannböden oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugte Packungen sind Blech- oder Gewebepackungen aus Metall oder Kunststoff. In der Regel sind 10 bis 30 theoretische Böden ausreichend. Die Wärmezufuhr erfolgt über innen- oder außenliegende Wärmetauscher herkömmlicher Bauart. Die Kondensation der Brüden am Kopf der Kolonne kann mit einem Kondensator herkömmlicher Bauart erfolgen. Das Kopfprodukt wird vorzugsweise wieder als Rücklauf der Kolonne zugeführt, das bevorzugte Rücklaufverhältnis liegt zwischen 1 bis 4 : 1.

Das isolierte Piperazin kann wiederum als Chips oder in anderer Form gewonnen und gelagert werden.

Die verbesserte Farbstabilität kann im Langzeittest überprüft werden. Da es jedoch vorteilhaft ist, schon möglichst bald nach der Isolierung den Erfolg der verbesserten Farbstabilität zu überprüfen, ist eine weitere Aufgabe der vorliegenden Erfindung die Bereitstellung eines Schnelltests.

Diese Aufgabe wird gelöst, in dem das isolierte Piperazin einem Temperschritt unterzogen wird und anschießend die Farbänderung, vorzugsweise mittels eines Farbzahltests, überprüft wird.

Diese Bewertung gelingt bereits kurz nach Isolierung, wenn man das Produkt dem erfindungsgemäßen Alterungsschnelltest unterzieht. Hierzu wird das Piperazin unter Luftausschluss beispielsweise bei 120°C über 4h getempert und anschließend seine Farbzahl in methanolischer oder wässriger Lösung bestimmt. Als Vergleich für das Langzeitverhalten kann auch die Farbzahl einer frisch angesetzten Piperazinlösung bestimmt werden.

Die Dauer des Temperschrittes und die zu haltende Temperatur können variiert werden. Sie sollten im Bereich des Schmelzpunktes von Piperazin liegen, und eine Dauer von einigen Stunden hat sich als zweckmäßig erwiesen. Das Auffinden geeigneter Temperaturwerte und Temperdauern erschließen sich dem Fachmann in naheliegender Weise.

Bei dem Farbzahltest erfolgt die Bestimmung der Farbqualität einer Verbindung generell durch Messung der Transmission eingestrahlten Lichtes. Hierzu wird die Schmelze oder eine Lösung einer bestimmten Konzentration in einer Küvette bekannter Schichtdicke mit einem Lichtstrahl einer definierten Wellenlänge durchstrahlt. Der Prozentsatz der durchgelassenen Lichtenergie ergibt bei gegebener Wellenlänge eine definierte Farbzahl. Gebräuchlich für schwachgefärbte Lösungen ist die Apha Farbscala.

Die Farbzahlmessung erfolgt in einem zuvor auf den mit dem verwendeten Wasser auf den Nullpunkt geeichten Spektrometer in einer 50-mm-Kunststoffküvette. Das Piperazin wird in pulverisierter Form eingewogen, wobei darauf zu achten ist, dass keine übermäßige Erwärmung durch Reibung eintritt. Auch muss der ganze Vorgang zügig in einem gut abgesaugten Ort durchgeführt werden, um Wassereintrag aufgrund der Hygroskopie des Piperazins gering zu halten.

Unter Anwendung dieser einfachen Analytik wird bestätigt, dass spezifikationsgerechtes Piperazin, welches als Chips aus der Schmelze der destillativen Aufarbeitung isoliert wird, nach der Temperung eine deutlich schlechtere Farbzahl aufweist als vor der Behandlung.

"Spezifikationsgerecht" bedeutet im Zusammenhang mit der vorliegenden Erfindung, dass das Piperazin die handelsüblichen Anforderungen an Reinheit, Höchstwassergehalt und Farbzahl erfüllt. Ein besonders hoher Maßstab für spezifikationsgerechtes Piperazin ist beispielsweise eine Reinheit von > 99,9% (GC), Wassergehalt < 1% und eine Apha Farbzahl ≤ 30.

Wiederum wird gefunden, dass Piperazin, welches nach destillativer Auftrennung zunächst durch Einleiten des Kolonnenaustrages in Wasser als 65%ige wässrige Lösung isoliert und anschließend im erfindungsgemäßen Verfahren destillativ getrocknet wird, eine deutlich bessere Farbstabilität im Alterungsschnelltest aufweist. Diese Ergebnisse werden in nachfolgenden Langzeitlagertests bestätigt.

Zudem wird gefunden, dass Piperazin, welches zunächst als Chips-Ware isoliert und erst nach Lagerung im spezifikationsgerechtem Zustand in wässrige Lösung überführt wird, durch anschließende destillative Trockung nach dem erfindungsgemäßen Verfahren ebenfalls im Alterungsschnelltest eine verbesserte Farbstabiltiät zeigt. Auch diese Ergebnisse werden in Langzeittests bestätigt.

Ferner wird gefunden, dass Piperazin, welches als Chips-Ware spezifikationsgerecht isoliert, durch längere Lagerung zwar chemisch spezifikationsgerecht aber vergilbt in wässrige Lösung überführt wird, durch anschließende destillative Trockung nach dem erfindungsgemäßen Verfahren ebenfalls im Alterungsschnelltest die Farbstabiltiät von frisch behandeltem Piperazin zeigt. Auch diese Ergebnisse werden in Langzeittests bestätigt.

Die vorliegende Erfindung wird anhand des nachfolgenden Beispiels näher erläutert.

### Beispiel

### Entwässerung von wässriger Piperazin Lösung

Eine wässrige Piperazin - Lösung wird in einem Vorratsbehälter von 20 Liter bei einer Temperatur von 65 °C bereitgestellt. Die Konzentration des Piperazins in der wässrigen Lösung beträgt 68 Gew.%. Es wird eine Laborkolonne (DN 50) mit 20 Glockenböden aus Glas gewählt. Der Zulauf erfolgt auf Boden 13. Die kontinuierliche Destillation wird bei einem Kopfdruck von 1 bar durchgeführt, die Sumpftemperatur beträgt 150°C. Die Zulaufmenge beträgt 1100 g/h, im Sumpf werden 735 g/h Piperazin mit einer Reinheit von 99,9 Gew.-% abgezogen, am Kopf wird Wasser mit einem geringen Anteil Piperazin mit einer Menge von 365 g/h erhalten. Das Rücklaufverhältnis beträgt 2:1.

### Farbzahltest

Etwa ein Gramm der gepulverten Probe wird in eine Flasche mit zweiteiligem Schraubdeckel eingewogen. Die Einwaage wird notiert.

Zur Entfernung von Luft wird das Gefäß in einen mit Vakuum- und Argonanschluss versehenen Exsikkator je dreimal evakuiert und mit Argon belüftet; anschließend wird die Probe unter Argonstrom verschlossen. Luft in der Probe ist unbedingt zu vermeiden, da Sauerstoff farbauslösende Oxidationsprodukte unter Temperbedingungen bewirkt.

Zur Entfernung von Luft kann das Gefäß auch während 30 Minuten mit Stickstoff durchgeblasen werden.

Das Probengefäß wird vollständig in ein vorgeheiztes Ölbad (oder einem vorgeheizten Ofen) (120°C) eingestellt, und 4 h bei gleichbeibender Temperatur gelagert.

Nachdem die Probe abgekühlt ist, wird die zehnfache Masse Wasser zugegeben, um das Piperazin zu lösen.

Folgende Farbzahlwerte vor und nach der Temperung werden nach diesem Verfahren ermittelt:

| Wasserfreie Probe | Farbzahlwert vor Temperung | Farbzahlwert nach Temperung |
|---|---|---|
| Probe Piperazin, spezifikationsgerecht (Stand der Technik) | 24 Apha | 54 Apha |
| Probe Piperazin nach erfindungs-gemäßer Isolierung | 22 Apha | 25 Apha |

## Patentansprüche

1. Verfahren zur Verbesserung der Farbstabilität durch Nachreinigung von Piperazin, die Schritte umfassend
- Herstellen einer wässrigen Piperazin-Lösung durch Einleiten festen oder flüssigen Piperazins mit einem Reinheitsgrad von mindestens 90 Gew.-% in Wasser;
- Entfernen des Wassers durch Destillation;
- Isolierung des Piperazins mit einem vorgegebenen Höchstrestgehalt an Wasser.

2. Verfahren nach Anspruch 1, wobei das feste oder flüssige Piperazin aus der destillativen Abtrennung eines Ethylenamin-Produktmixes oder einer EDA/Piperazin-Fraktion erhalten wird.

3. Verfahren nach Anspruch 2, wobei das Piperazin direkt als Fraktionsaustrag in Wasser eingeleitet wird.

4. Verfahren nach einem der Ansprüche 1 bis 3, wobei das feste Piperazin als Chips-Ware vorliegt.

5. Verfahren nach einem der Ansprüche 1 bis 4, wobei das feste oder flüssige Piperazin aus dem MEOA Verfahren erhalten wird.

6. Verfahren nach einem der Ansprüche 1 bis 5, wobei das feste oder flüssige Piperazin einen Reinheitsgrad von > 95 Gew.-% aufweist.

7. Verfahren nach einem der Ansprüche 1 bis 6, wobei das isolierte Piperazin einen Höchstrestgehalt an Wasser von < 15 Gew.-% hat.

8. Verfahren nach einem der Ansprüche 1 bis 7, wobei das Wasser durch Kolonnendestillation über den Kollonenkopf entfernt wird und wobei das Piperazin aus dem Sumpf oder über einen Seitenabzug der Aufreinigungskolonne isoliert wird.

9. Verfahren nach Anspruch 8, wobei die Sumpftemperatur 130 - 170 °C beträgt, der Druck am Kolonnenkopf 0,6 bis 2 bar beträgt, die Kolonne über 10 bis 30 theoretische Böden verfügt und/oder wobei das Rücklaufverhältnis zwischen 1 bis 4 : 1 liegt.

10. Verfahren nach einem der Ansprüche 1 bis 9, wobei das isolierte Piperazin einem Temperschritt unterzogen und anschließend die Farbänderung überprüft wird.

## Claims

1. A process for improving the color stability by postpurifying piperazine, the steps comprising
- preparing an aqueous piperazine solution by introducing solid or liquid piperazine having a degree of purity of at least 90% by weight in water;
- removing the water by distillation;
- isolating the piperazine with a predefined maximum residual content of water.

2. The process according to claim 1, wherein the solid or liquid piperazine is obtained from the distillative removal of an ethylenamine product mix or an EDA/piperazine fraction.

3. The process according to claim 2, wherein the piperazine is introduced into water directly as a fraction effluent.

4. The process according to any of claims 1 to 3, wherein the solid piperazine is present as chip material.

5. The process according to any of claims 1 to 4, wherein the solid or liquid piperazine is obtained from the MEOA process.

6. The process according to any of claims 1 to 5, wherein the solid or liquid piperazine has a degree of purity of > 95% by weight.

7. The process according to any of claims 1 to 6, wherein the isolated piperazine has a maximum residual content of water of < 15% by weight.

8. The process according to any of claims 1 to 7, wherein the water is removed by column distillation via the top of the column and wherein the piperazine is isolated from the bottom or via a side draw of the purification column.

9. The process according to claim 8, wherein the bottom temperature is 130 - 170°C, the pressure at the top of the column is from 0.6 to 2 bar, the column has from 10 to 30 theoretical plates and/or the reflux ratio is between 1 : 1 and 4 : 1.

10. The process according to any of claims 1 to 9, wherein the isolated piperazine is subjected to a heat treatment step and the color change is subsequently tested.

## Revendications

1. Procédé pour l'amélioration de la stabilité de la couleur par post-purification de la pipérazine, les étapes comprenant
- préparation d'une solution aqueuse de pipérazine par introduction de pipérazine solide ou liquide, ayant un degré de pureté d'au moins 90 % en poids, dans de l'eau ;
- élimination de l'eau par distillation ;
- isolement de la pipérazine ayant une teneur résiduelle maximale préétablie en eau.

2. Procédé selon la revendication 1, dans lequel la pipérazine solide ou liquide est obtenue à partir du fractionnement par distillation d'un mélange de produits contenant de l'éthylène-amine ou une fraction EDA/pipérazine.

3. Procédé selon la revendication 2, dans lequel la pipérazine est introduite directement en tant que décharge de fraction dans l'eau.

4. Procédé selon l'une quelconque des revendications 1 à 3, dans lequel la pipérazine solide se trouve sous forme de produit en fragments.

5. Procédé selon l'une quelconque des revendications 1 à 4, dans lequel la pipérazine solide ou liquide est obtenue à partir du procédé MEOA.

6. Procédé selon l'une quelconque des revendications 1 à 5, dans lequel la pipérazine solide ou liquide présente un degré de pureté de > 95 % en poids.

7. Procédé selon l'une quelconque des revendications 1 à 6, dans lequel la pipérazine isolée a une teneur résiduelle maximale en eau de < 15 % en poids.

8. Procédé selon l'une quelconque des revendications 1 à 7, dans lequel l'eau est éliminée par distillation sur colonne, par la tête de la colonne, et dans lequel la pipérazine est isolée à partir du pied ou par un conduit de décharge latéral de la colonne de purification.

9. Procédé selon la revendication 8, dans lequel la température au pied est de 130 - 170 °C, la pression à la tête de la colonne est de 0,6 à 2 bars, la colonne est dotée de 10 à 30 plateaux théoriques et/ou dans lequel le taux de reflux est compris entre 1 et 4 : 1.

10. Procédé selon l'une quelconque des revendications 1 à 9 dans lequel on soumet la pipérazine isolée à une étape de traitement thermique et ensuite on teste le changement de couleur.
